# EUROPEAN PATENT APPLICATION

(11) **EP 3 978 128 A1**
(43) Date of publication of application: **06.04.2022**
(21) Application number: 21199839.8
(22) Date of filing: 29.09.2021
(51) Int. Cl.: B01L 3/00, B01L 7/00, G01N 1/40, G01N 27/26, C12Q 1/68

(54) **NUCLEIC ACID DETECTION KIT AND NUCLEIC ACID DETECTION DEVICE**

(30) Priority: 30.09.2020 US 202063085385 P; 30.09.2020 US 202063085368 P; 30.06.2021 CN 202110735245
(71) Applicant: iCare Diagnostics International Co. Ltd., New Taipei 236 (TW)
(72) Inventor: CHANG, Chia-Hsin, 236 New Taipei (TW); WU, Chang-Chin, 236 New Taipei (TW); CHIU, Peng-Yu, 236 New Taipei (TW); HUANG, Ching-Chu, 236 New Taipei (TW); HSU, Wei-Hua, 236 New Taipei (TW)
(74) Representative: Zaboliene, Reda

(57) **Abstract**

A nucleic acid detection kit includes a kit body, a detection chip disposed in the kit body, and an electrophoresis box disposed outside of the kit body. The detection chip comprises a channel and is connected to the electrophoresis box. A microbead of testable material in solution undergoes a PCR amplification reaction, an electrophoretic process, and is fluoresced for highlighting, images of elements made fluorescent can be taken and displayed. A nucleic acid detection device which can receive the nucleic acid detection kit is also disclosed. The nucleic acid detection device has a simple structure suitable for home use, being portable, flexible, and convenient.

## Description

The subject matter relates to nucleic acid detection devices, and more particularly, to a nucleic acid detection kit and a nucleic acid detection device with the nucleic acid detection kit.

### BACKGROUND

Molecular diagnosis, morphological detection, and immunological detection are mostly carried out in laboratories. The detection process includes performing a PCR amplification reaction in a large or a medium-sized detection equipment to acquire an amplified product. Then, the amplified product is manually transferred to an electrophoretic process equipment for an electrophoretic process. Finally, a result of the electrophoretic process is manually transferred to a fluorescence analyzer to obtain an image of fluorescence. However, such processes are time-consuming, inefficient, and inflexible, and the device is not portable. The analysis cannot be carried out anytime and anywhere.

### SUMMARY

The present disclosure provides a nucleic acid detection kit including a kit body. The nucleic acid detection kit further includes a detection chip disposed in the kit body and an electrophoresis box disposed outside of the kit body. The detection chip includes a first cover plate, a spacer layer, and a second cover plate, two opposite surfaces of the spacer layer are in contact with the first cover plate and the second cover plate respectively, the first cover plate, the spacer layer, and the second cover plate cooperatively define a channel configured for carrying a microbead, the detection chip is connected to the electrophoresis box, the microbead undergoes a PCR amplification reaction to obtain a mixed microbead in the channel, the mixed microbead undergoes an electrophoretic process in the electrophoresis box.

In some embodiments, the detection chip further includes a driving circuit, a first dielectric layer, a conductive layer, and a second dielectric layer, the driving circuit is disposed on a surface of the first cover plate close to the second cover plate, the first dielectric layer is disposed on a side of the driving circuit close to the second cover plate, the conductive layer is disposed on a surface of the second cover plate close to the first cover plate, the second dielectric layer is disposed on a side of the conductive layer close to the first cover plate, each of the driving circuit and the conductive layer is configured to electrically connect to a power supply, the first dielectric layer and the second dielectric layer cooperatively define the channel.

In some embodiments, the driving circuit includes at least one PCR amplification area, the kit body defines at least one heating hole, the at least one heating hole corresponds to a corresponding one of the at least one PCR amplification area, the detection chip exposes through the at least one heating hole.
In some embodiments, the kit body includes a first housing and a second housing, and each of the first housing and the second housing defines the at least one heating hole.

In some embodiments, the driving circuit includes a plurality of driving electrodes disposed in an array, and each of the plurality of driving electrodes is electrically connected to a power supply.

In some embodiments, the nucleic acid detection kit further includes a control board, each of the plurality of driving electrodes and the conductive layer are electrically connected to the control board, the control board is disposed on a surface of the first cover plate close to the second cover plate, and the control hoard is disposed outside of the channel, the kit body further includes a connecting opening, the control board exposes through the connecting opening.

In some embodiments, the driving circuit further includes a sample adding area, a reagent storage area, and a solution outlet area, and the solution outlet area is connected to the electrophoresis box.

In some embodiments, the nucleic acid detection kit further includes a reagent capsule disposed in the reagent storage area, the reagent capsule extends out of the second cover plate, the kit body defines a reagent groove corresponding to the reagent storage area, and the reagent capsule is received in the reagent groove.

In some embodiments, the electrophoresis box includes an electrophoretic body, two electrophoretic electrodes, and a gel medium, the two electrophoretic electrodes are disposed on two ends of the electrophoretic body, the two electrophoretic electrodes are opposite to each other, the gel medium is disposed in the electrophoretic body, a liquid injection slot is disposed on an end of the gel medium, each of the two electrophoresis electrodes is configured to electrically connect to a power supply, the first cover plate defines an outlet, and the liquid injection slot is connected to the outlet.

In some embodiments, the nucleic acid detection kit further includes a first porous adsorption block disposed in the liquid injection slot, the first porous adsorption block is configured to carry a wetting liquid, one end of the first porous adsorption block extends into a bottom of the liquid injection slot, and the other end of the first porous adsorption block extends into the outlet, the first porous adsorption block is flush with or protrude from an opening of the outlet closed to the channel.

In some embodiments, the nucleic acid detection kit further includes a second porous adsorption block and an adsorption pipe, the second porous adsorption block is disposed in the liquid injection slot, one end of the adsorption pipe is inserted into the second porous adsorption block, and the other end of the adsorption pipe is flush with or protruding from an opening of the outlet closed to the channel, the second porous adsorption block is configured to carry a wetting liquid, the wetting liquid further extends into and fills the adsorption pipe.

The present dis24closure further provides a nucleic acid detection device including a host. The nucleic acid detection device further includes the above nucleic acid detection kit. A detection kit mounting area is disposed on the host, the nucleic acid detection kit is detachably disposed in the detection kit mounting area.

In some embodiments, the nucleic acid detection device further includes a host heating unit and a host connector, the host heating unit and the host connector are disposed in the detection kit mounting area, each of the detection chip and the electrophoresis box is electrically connected to the host connector, the host heating unit is connected to a surface of the detection chip and is configured to heat the nucleic acid detection kit.

the nucleic acid detection device provided by the present disclosure integrates the PCR amplification reaction and the electrophoretic process of nucleic acid into in a single equipment through the cooperation of the host and the nucleic acid detection kit. Thus, the nucleic acid detection device has a simple structure, which is portable, flexible, and convenient, and can be used at home.

### BRIEF DESCRIPTION OF THE DRAWINGS

Implementations of the present technology will now be described, by way of example only, with reference to the attached figures.
FIG. 1 is a front, top perspective view of an embodiment of a nucleic acid detection kit according to the present disclosure.
FIG. 2 is a rear, bottom perspective view of the nucleic acid detection kit according to the present disclosure,
FIG. 3 is an exploded diagrammatic view of the nucleic acid detection kit according to the present disclosure.
FIG. 4 is a rear, bottom perspective view of an embodiment of a nucleic acid detection kit without a kit body according to the present disclosure.
FIG. 5 is a diagrammatic view of an embodiment of a detection chip of the nucleic acid detection kit according to the present disclosure.
FIG. 6 is a cross-sectional view of the detection chip according to the present disclosure.
FIG. 7 is a diagrammatic view of an embodiment of a driving circuit of a detection chip according to the present disclosure.
FIG. 8 is a diagrammatic view of an embodiment of an electrophoresis box of the nucleic acid detection kit according to the present disclosure.
FIG. 9 is a diagrammatic view of an embodiment of a connective structure connecting a detection chip and an electrophoresis box according to the present disclosure.
FIG. 10 is a diagrammatic view of another embodiment of a connective structure connecting a detection chip and an electrophoresis box according to the present disclosure.
FIG. 11 is a diagrammatic view of an embodiment of a nucleic acid detection device according to the present disclosure.
FIG. 12 is a cross-sectional view the nucleic acid detection device according to the present disclosure.
FIG. 13 is an image of fluorescence generated by the nucleic acid detection kit according to the present disclosure.

### DETAILED DESCRIPTION

It will be appreciated that for simplicity and clarity of illustration, where appropriate, reference numerals have been repeated among the different figures to indicate corresponding or analogous components. In addition, numerous specific details are set forth in order to provide a thorough understanding of the embodiments described herein. However, it will be understood by those of ordinary skill in the art that the embodiments described herein can be practiced without these specific details. In other instances, methods, procedures, and components have not been described in detail so as not to obscure the related relevant feature being described. Also, the description is not to be considered as limiting the scope of the embodiments described herein. The drawings are not necessarily to scale, and the proportions of certain parts may be exaggerated to better illustrate details and features of the present disclosure.

The term "comprising," when utilized, means "including, but not necessarily limited to"; it specifically indicates open-ended inclusion or membership in the so-described combination, group, series, and the like.

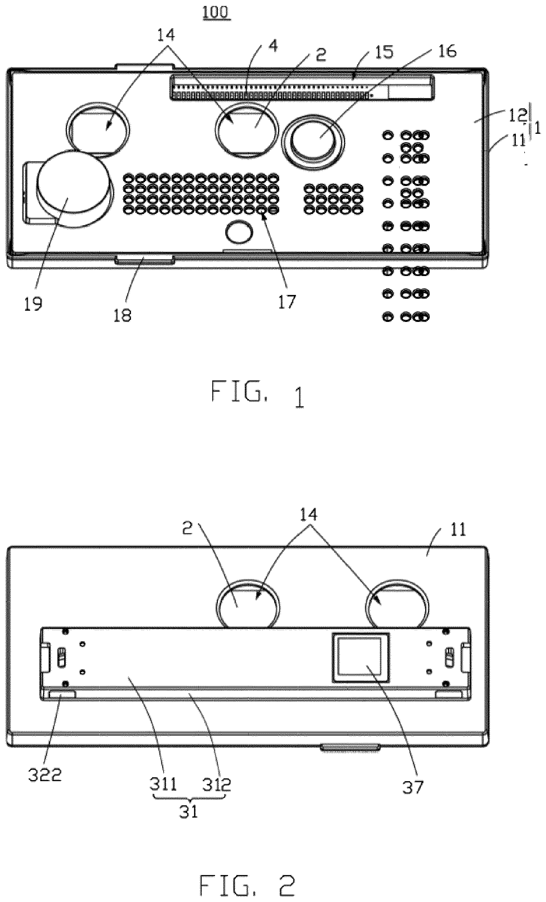

FIGS. 1 to 7 illustrate a nucleic acid detection kit 100, which includes a kit body 1, a detection chip 2, and an electrophoresis box 3. The detection chip 2 is disposed in the kit body 1. The electrophoresis box 3 is disposed outside of the kit body 1. The detection chip 2 is connected to the electrophoresis box 3. The detection chip 2 is used to perform a PCR amplification reaction. The electrophoresis box 3 is used to perform analysis by an electrophoretic process. The detection chip 2 includes a first cover plate 21, a spacer layer 22, and a second cover plate 23. Two opposite surfaces of the spacer layer 22 are in contact with the first cover plate 21 and the second cover plate 23 respectively. The first cover plate 21, the spacer layer 22, and the second cover plate 23 cooperatively define a channel 5 for carrying a testable material in solution ("solution") to be detected. The solution in the channel 5 is in a form of microbead "a". The microbead "a" may undergo the PCR amplification reaction to obtain a mixed microbead "b". The mixed microbead "b" is driven to enter the electrophoresis box 3 to undergo the electrophoretic process. An image collection unit (not shown) acquires an image of fluorescence within the electrophoresis box 3. The nucleic acid detection kit 100 integrates with the detection chip 2 and the electrophoresis box 3, which has a small size, and is suitable for a portable use. After the PCR amplification reaction is completed, the electrophoretic process can be carried out automatically. The two processes are performed in a single equipment, and the sampling accuracy is precise. Thus, the detection process is efficient and flexible.

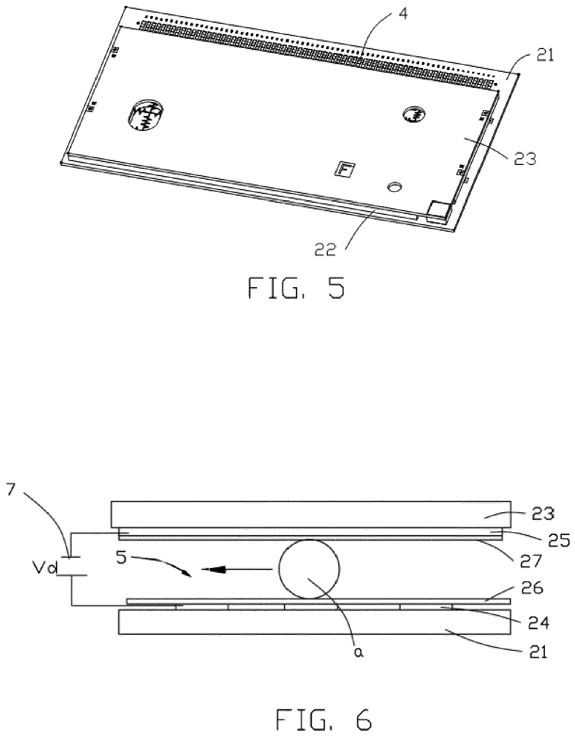

Referring to FIG. 6, the detection chip 2 further includes a driving circuit 24, a first dielectric layer 26, a conductive layer 25, and a second dielectric layer 27. The driving circuit 24 is disposed on a first surface of the first cover plate 21 close to the second cover plate 23. The first dielectric layer 26 is disposed on a side of the driving circuit 24 close to the second cover plate 23. The conductive layer 25 is disposed on a first surface of the second cover plate 23 close to the first cover plate 21. The second dielectric layer 27 is disposed on a side of the conductive layer 25 close to the first cover plate 21. The driving circuit 24 and the conductive layer 25 are electrically connected to a power supply 7. The microbead "a" can be driven to move along a path in the channel 5 by energizing or de-energizing a circuit between the driving circuit 24 and the conductive layer 25.

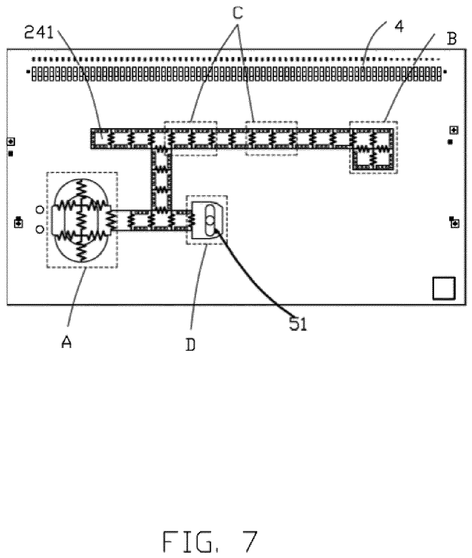

Referring to FIGS. 6 and 7, the driving circuit 24 includes a plurality of driving electrodes 241 disposed in an array. The nucleic acid detection kit 100 further includes a control board 4. The driving electrodes 241 and the conductive layer 25 are electrically connected to the control board 4. The control board 4 is electrically connected to the power supply 7. In an embodiment, the control board 4 is disposed on a surface of the first cover plate 21 close to the second cover plate 23, and outside of the channel 5.

In an embodiment, the driving circuit 24 is a thin film transistor (TFT) driving circuit. The microbead "a" has some conductivity in itself, and can be driven by circuits applying dielectric wetting and de-wetting between the driving electrodes 241 and the conductive layer 25 to move along the path in the channel 5 due to dielectric welting principle (EWOD). Due to the EWOD principle, one of the circuits between one of the driving electrodes 241 and the conductive layer 25 can be selectively energized to change wetting characteristics between the microbead "a" and the first dielectric layer 26 and between the microbead "a" and the second dielectric layer 27, so as to move the microbead "a" along the path. Referring to FIG. 6, the driving electrodes 241 include a driving electrode "I", a driving electrode "H", and a driving electrode "G". The microbead "a" moves on the driving electrode "I", the driving electrode "H", and the driving electrode "G". When the microbead "a" is on the driving electrode "H", a voltage "Vd" is applied between the driving electrode "G" and the conductive layer 25, and the driving electrode "H" is disconnected from the conductive layer 25. At this time, the wetting characteristics between the microbead "a" and the first dielectric layer 26, and between the microbead "a" and the second dielectric layer 27 are changed, so that a liquid-solid contact angle between the driving electrode "H" and microbead "a" becomes larger, and a liquid-solid contact angle between the driving electrode "G" and microbead "a" becomes smaller, to promote the movement of the microbead "a" from the driving electrode "H" to the driving electrode "G".

In an embodiment, the first dielectric layer 26 and the second dielectric layer 27 are insulated and are hydrophobic. On the one hand, the first dielectric layer 26 and the second dielectric layer 27 have the characteristics of insulation and hydrophobicity, and on the other hand, the first dielectric layer 26 and the second dielectric layer 27 can make the microbead "a" move smoothly along the path, avoiding breakage and fragmentation of the liquid microbead "a" during movement.

In an embodiment, each of the first dielectric layer 26 and the second dielectric layer 27 may be, but is not limited to, a polytetrafluoroethylene coating.

Referring to FIG. 7, in an embodiment, the driving circuit 24 may be formed on the first surface of the first cover plate 21 by metal etching or electroplating.

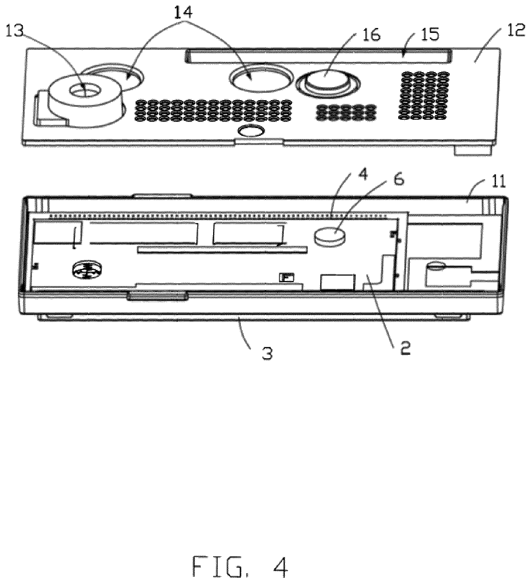

Referring to FIGS. 4 and 7, the driving circuit 24 can be divided into a plurality of areas according to their different purposes, including a sample adding area "A", a reagent storage area "B", a plurality of PCR amplification areas "C", and a solution outlet area "D". The detection chip 2 corresponding to the sample adding area "A" defines a detection kit sampling groove 6. The detection chip sampling groove 6 can correspond to the sampling port 13 on the second cover plate 23. The microbead "a" is added in the sampling area "A" through the sampling port 13. The reagent storage area "B" is used to store fluorescent reagents (such as fluorescent dyes or fluorescent probes). The microbead "a" undergoes PCR amplification reaction in the PCR amplification areas "C" to form an amplified product. The amplified product is mixed with a fluorescent reagent in the reagent storage area "B" to form the mixed microbead "b". The solution outlet area "D" is connected to the electrophoresis box 3. The mixed microbead "b" is driven to enter the electrophoresis box 3 through the solution outlet area "D". The number of the PCR amplification areas "C" can be determined according to an actual requirement

After the microbead "a" enters the sampling area "A", the microbead "a" moves to the PCR amplification areas "C" and undergoes the PCR amplification reaction to form an amplified product. When the PCR amplification reaction is completed, the amplified product is moved to the reagent storage area "B" and mixed with the fluorescent reagent to obtain the mixed microbead "b". The mixed microbead "b" then enters the electrophoresis box 3 through an outlet 51 in the solution outlet area "D" and undergoes an electrophoretic process.

In order to mix the amplified product and the fluorescent reagent more evenly, the mixed microbead "b" is moved back and forth several times in the PCR amplification area "C. A mixing area (not shown) can also be set separately in the driving circuit 24 to mix the amplified product and the fluorescent reagent.

In an embodiment, the number of the PCR amplification areas "C" is two, or three, or more.

In an embodiment, there are two PCR amplification areas "C". One of the PCR amplification areas "C" has a temperature range from 90 °C to 105 °C. The other one of the PCR amplification areas "C" has a temperature range from 40 °C to 75 °C.

In yet another embodiment, the number of the PCR amplification areas "C" is three. One of the three PCR amplification areas "C" has a temperature range from 90 °C to 105 °C. A second one of the three PCR amplification areas "C" has a temperature range from 68 °C to 75 °C. A third one of the three PCR amplification areas "C" has a temperature range from 40 °C to 65 °C.

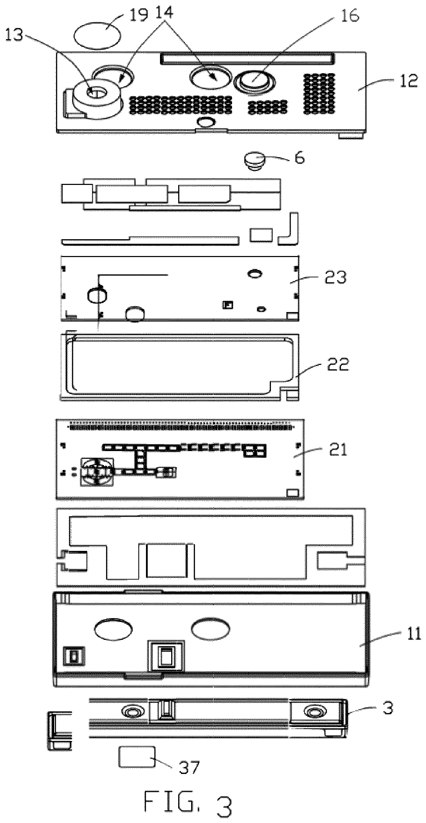

Referring to FIGS. 3, 4 and 7, a reagent capsule 6 is placed in the reagent storage area "B". The fluorescent reagent (such as a fluorescent dye or a DNA probe) is received in the reagent capsule 6. The reagent capsule 6 extends out of the second cover plate 23. The kit body 1 defines a reagent groove 16. The reagent groove 16 corresponds to the reagent storage area "B", and the reagent capsule 6 is received in the reagent groove 16. The reagent capsule 6 can be made molten by a first external heater (not shown) during the PCR amplification reaction. Then the fluorescent reagent may flow out from the reagent capsule 6 to mix with the amplified product. Thus, there is no need to add fluorescent reagent in the detection chip 2 separately.

In yet another embodiment, the fluorescent reagent can also be placed in the reagent storage area "B" in advance. Thus, there is no need to add fluorescent reagent in the detection chip 2 separately.

In an embodiment, silicone oil may be injected into the channel 5 after the detection chip 2 is assembled, and the microbead "a" is driven to move in the silicone oil.

Referring to FIGS. 5 and 6, in an embodiment, the first cover plate 21 and the second cover plate 23 are glass plates. The spacer layer 22 is a frame with adhesive on both sides, which is connected to edges of the first cover plate 21 and the second cover plate 23 to define the channel 5. A volume of the channel 5 can be adjusted by changing a thickness of the spacer layer 22 according to an actual demand.

Referring to FIGS. 1 to 3, the kit body 1 includes a first housing 11, a second housing 12, a sampling port 13 disposed on the second housing 12, a connecting opening 15 disposed on the second housing 12, and at least one heating hole 14. The first housing 11 and the second housing 12 are connected together to define a receiving cavity (not shown in the figures). The detection chip 2 is disposed in the receiving cavity. The electrophoresis box 3 is disposed on a side of the first housing 11 away from the second housing 12. The heating hole 14 is disposed on the first housing 11 and / or the second housing 12. The sampling port 13 can correspond to the sample adding area "A" of the detection chip 2, and the microbead "a" can be added into the detection chip 2 through the sampling port 13. The connecting opening 15 corresponds to the control board 4. The control board 4 is exposed through the connecting opening 15. The power supply 7 can extend into the connecting opening 15 and connect to the control board 4. The heating hole 14 corresponds to the PCR amplification areas "C". A surface of the detection chip 2 corresponds to a PCR amplification area "C" and is exposed through the heating hole 14. A second external heater (not shown) can extend in one heating hole 14 and connect to the detection chip 2 to heat the PCR amplification area "C".

In an embodiment, the second housing 12 defines the reagent groove 16. The first external heater is disposed on a side of the second housing 12 away from the first housing 11 and connected to an outer surface of the reagent groove 16. The first external heater is used to heat the reagent groove 16 to render the reagent capsule 6 molten in the reagent groove 16.

In an embodiment, each of the first housing 11 and the second housing 12 defines at least one heating hole 14. Each of the heating hole(s) 14 corresponds to one corresponding second external heater. Two opposite surfaces of the detection chip 2 can be heated together, and the temperatures of the PCR amplification areas "C" are even. The number of the heating hole 14 is set according to the number of the PCR amplification area "C".

In an embodiment, the number of the heating hole 14 is twice that of the PCR amplification area "C". When there are two PCR amplification areas "C", the number of the heating holes 14 is four.

In an embodiment, the first housing 11 and the second housing 12 are connected to each other through an adhesive.

In yet another embodiment, the first housing 11 and the second housing 12 are assembled together by a latching arrangement or snapped together. The first housing 11 and the second housing 12 are further fastened together by screws to increase a connection strength therebetween.

Referring to FIG. 1, 3, and 11, the kit body 1 further includes two convex blocks 18. The two convex blocks 18 are disposed opposite to each other and are disposed on two sidewalls of the kit body 1. The two convex blocks 18 are used to facilitate the insertion and removal of the nucleic acid detection kit 100 in a nucleic acid detection device 200.

In an embodiment, the second housing 12 defines several through holes 17. The through holes 17 are used to dissipate the heat in the kit body 1.

In an embodiment, a covering film 19 is disposed on the sampling port 13 to seal the sampling port 13.

Referring to FIG. 2 to 4, 8, and 9, the electrophoresis box 3 includes an electrophoretic body 31, two electrophoretic electrodes 32, a gel medium 33, and a liquid injection slot 34. One electrophoretic electrode 32 is disposed on a first end of the electrophoretic body 31. The other electrophoretic electrode 32 is disposed on a second end of the electrophoretic body 31 away from the first end. The two electrophoretic electrodes are opposite with each other. The electrophoretic body 31 defines an electrophoretic groove 313, the gel medium 33 is disposed in the electrophoretic body 313. The liquid injection slot 34 is disposed on an end of the gel medium 33. Each of the two electrophoresis electrodes 32 is electrically connected to the power supply 7. The liquid injection slot 34 penetrates the first housing 11 and is connected to the outlet 51 to make a connection between the detection chip 2 and the electrophoresis box 3. The mixed microbead "b" on the solution outlet area "D" may enter the gel medium 33 through the outlet 51 and the liquid injection slot 34, thereby presenting itself for the electrophoretic process.

Referring to FIGS. 2, 3, and 8, the electrophoretic body 31 is disposed on a side of the first cover plate 21 away from the second cover plate 23. An opening of the electrophoretic body 31 faces the first cover plate 21. The electrophoretic body 31 includes a transparent substrate 311 and a plurality of sidewalls 312 connected to the transparent substrate 311. The transparent substrate 311 and the sidewalls 312 cooperatively form the electrophoretic groove 313. The transparent substrate 311 is a transparent plate, and an image attention unit (not shown) can collect an image of fluorescence within the electrophoresis box 3 through the transparent substrate 311.

In an embodiment, ends of the sidewalls 312 away from the transparent substrate 311 are connected to the surface of the first cover plate 21, thereby, the electrophoretic body 31 is covered by the first cover plate 21, With the above configuration, the electrophoresis box 3 can better connect to the detection chip 2, which facilitates the transfer of the mixed microbead "b" from the detection chip 2 to the electrophoresis box 3. The nucleic acid detection kit 100 integrates with the detection chip 2 and the electrophoresis box 3, which has a small size, and is suitable for the nucleic acid detection device 200.

In an embodiment, a sealing rubber ring (not shown) is disposed between the sidewall 312 and the first cover plate 21 to improve sealing of the electrophoresis box 3.

In yet another embodiment, an electrophoresis cover plate (not shown) is disposed on the opening of the electrophoretic body 31. The liquid injection slot 34 penetrates the electrophoresis cover plate and the first housing 11, and is connected to the outlet 51.

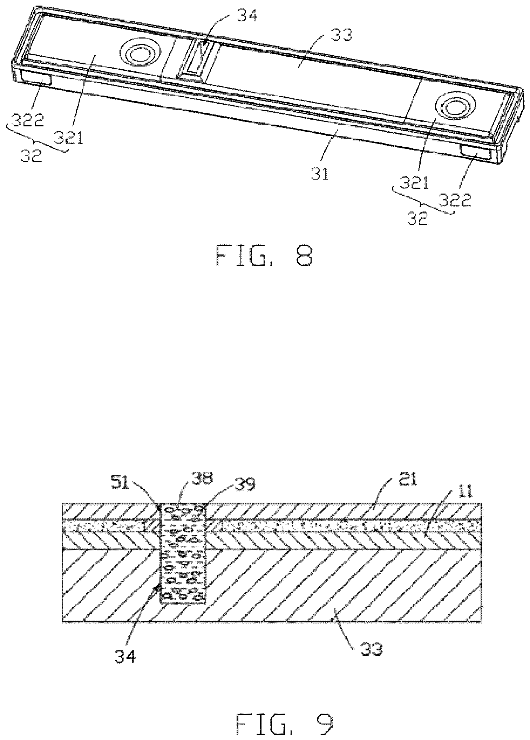

Referring to FIG. 8, the electrophoretic body 31 further includes a plurality of clamping portions (not shown) disposed on the transparent substrate 311. The clamping portions fix the gel medium 33 and prevent the gel medium 33 from moving out of position, thereby guaranteeing the accuracy of the electrophoretic process.

In an embodiment, the gel medium 33 is substantially cubic.

In an embodiment, the transparent substrate 311 is a transparent glass plate, and the image of fluorescence of the electrophoresis box 3 can be observed on a side of the transparent substrate 311 away from the gel medium 33.

In an embodiment, there are four clamping portions. Four clamping portions are disposed outside of the gel medium 33 to fix the gel medium 33 in place.

In an embodiment, the gel medium 33 may be, but is not limited to, an agar gel, and an agarose gel.

Referring to FIG. 7, 8 and 9, in an embodiment, for smooth entry of the mixed microbead "b" into the electrophoresis box 3, a first porous adsorption block 38 is disposed in the liquid injection slot 34. The first porous adsorption block 38 carries a wetting liquid 39. One end of the first porous adsorption block 38 extends to the bottom of the liquid injection slot 34, and the other end extends to the outlet 51. The first porous adsorption block 38 is flush with or protruding from an opening of the outlet 51 close to the channel 5. When the mixed microbead "b" moves to the liquid outlet area "D", it may contact the first porous adsorption block 38, then the mixed microbead "h" dissolves in the wetting liquid 39 in the first porous adsorption block 38 and flows to the bottom of the liquid injection slot 34 along the first porous adsorption block 38. The first porous adsorption block 38 adsorbs a small amount of wetting liquid 39, which reduces the amount of the wetting liquid in the electrophoretic body 31. The electrophoresis box 3 with a small amount of wetting liquid 39 provided by the present disclosure is a dry electrophoresis process. With the above configuration, it simplifies the assembly process and assembly difficulty of the nucleic acid detection kit 100, and facilitates the entry of the mixed microbead "b" into the liquid injection slot 34.

In an embodiment, the first porous adsorption block 38 may be made of, but is not limited to, a sponge, or other porous material.

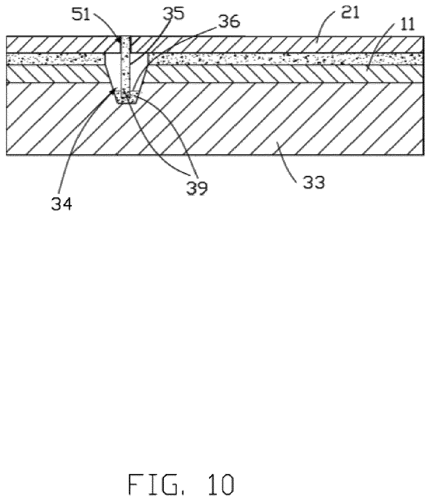

Referring to FIG. 8 and 10, in yet another embodiment, a second porous adsorption block 36 is disposed in the liquid injection slot 34. The second porous adsorption block 36 carries the wetting liquid 39. An adsorption pipe 52 is disposed in the outlet 51. One end of the adsorption pipe 52 is inserted into the second porous adsorption block 36, the other end is flush with or protruding from the opening of the outlet 51 close to the channel 5. The wetting liquid 39 in the second adsorption block 36 extend into and fills the adsorption pipe 52. When the mixed microbead "b" moves to the liquid outlet area "D", the mixed microbead "b" makes contact with the wetting liquid 39 in the adsorption tube 52 and dissolves in the wetting solution 39, and then enters the liquid injection slot 34 along the adsorption pipe 52 and the second porous adsorption block 36. By cooperation of the adsorption pipe 52 and the second adsorption block 36, the mixed microbead "b" enters smoothly into the electrophoresis box 3 from the channel 5. The assembly process of the nucleic acid detection kit 100 is simplified, and the assembly difficulty is low.

In yet another embodiment, a buffer is added into the liquid injection slot 34, and the buffer is connected the silicone oil to form a continuous flow and ensure that the mixed microbead "b" enters the electrophoresis box 3 smoothly and completely.

Referring to FIG. 4, each of the electrophoresis electrodes 32 includes an electrode body 321 and an electrode sheet 322 electrically connected to the electrode body 321. Two electrode sheets 322 are disposed on one sidewall 312 of the electrophoretic body 31 to electrically connect to the power supply 7.

In an embodiment, the electrode body 321 may be made of, but is not limited to, metal, a composite of metal and anionic resin, or a composite of metal and cationic resin.

In an embodiment, the electrode body 321 may be made of metal. The metal is copper.

Referring to FIG. 2, the electrophoresis box 3 further includes an electronic identification code 37 (such as a QR code) attached on an outer surface of the transparent substrate 311 of the electrophoretic body 31. When collecting the image of fluorescence, the image collection unit records the electronic identification code 37 to classify the image of fluorescence.

A method for using the nucleic acid detection kit 100 to perform the PCR amplification reaction and the electrophoretic process includes followings steps.

At step one, referring to FIG. 1, a solution to be detected containing a nucleic acid sample is injected into the detection chip 2 through the sampling port 13. The solution in the detection chip 2 is in the form of microbead "a" by pressure control.

At step two, referring to FIG. 7, the microbead "a" is driven by circuits applying dielectric wetting and de-wetting between the driving electrodes 241 and the conductive layer 25 to move along the flow path in the channel 5 to undergo the PCR amplification reaction to form an amplified product. The number of the PCR amplification areas "C" is two. One PCR amplification area "C" has a temperature range from 90 °C to 105 °C. Another PCR amplification area "C" has a temperature range from 40 °C to 75 °C.

In an embodiment, the PCR amplification reaction includes the following four steps. At step one, a thermal denaturation of the microbead "a" is performed at a temperature range from 90 °C to 105 °C for 15 min to 25 min. At step two, an RT reverse transcription of the microbead "a" after the thermal denaturation is performed at a temperature range from 45 °C to 60 °C for 5 min to 15 min. At step three, the microbead "a" after the RT reverse transcription is heated at a temperature range from 90 °C to 100 °C for 1 min to 5 min. At step four, the microbead "a" is heated at a temperature range from 90 °C to 100 °C for 20 seconds to 50 seconds, then heated at a temperature range from 55 °C to 65 °C for 40 seconds to 60 seconds. The fourth step is repeated in a range from 35 cycles to 50 cycles (such as 40 cycles) to form an amplified product. In an embodiment, a temperature sensor and a time relay are used to sense the temperature and the heating time.

In yet another embodiment, the PCR amplification reaction includes the following four steps. At step one, a thermal denaturation of the microbead "a" is performed at a temperature range from 90 °C to 105 °C for 3 min to 8 min. At step two, an amplification reaction of the microbead "a" after the thermal denaturation is performed at a temperature range from 45 °C to 60 °C for 3 min to 8 min. At step three, the microbead "a" after the amplification reaction is heated at a temperature range from 90 °C to 100 °C for 3 min to 8 min. At step four, the microbead "a" is heated at a temperature range from 90 °C to 100 °C for 3 seconds to 8 seconds, then heated at a temperature range from 50 °C to 65 °C for 10 seconds to 20 seconds, then heated at a temperature range from 68 °C to 75 °C for 10 seconds to 20 seconds. The fourth step is repeated in a range from 35 cycles to 50 cycles to form an amplified product.

In an embodiment, at step one, a thermal denaturation of the microbead "a" is performed at a temperature range from 90 °C to 97 °C for 3 min to 5 min. At step two, an amplification reaction of the microbead "a" after the thermal denaturation is performed at a temperature range from 55 °C to 60 °C for 3 min to 5 min. At step three, the microbead "a" after the amplification reaction is heated at a temperature range from 95 °C to 97 °C for 3 min to 8 min. At step four, the microbead "a" is heated at a temperature range from 95 °C to 97 °C for 3 seconds to 5 seconds, then heated at a temperature range from 55 °C to 60 °C for 15 seconds to 20 seconds, then heated at a temperature range from 70 °C to 72 °C for 15 seconds to 20 seconds. The fourth step is repeated in a range from 43 cycles to 45 cycles (such as 45 cycles) to form an amplified product

At step three, referring to FIGS. 4 and 7, the amplified product moves to the reagent storage area "B". The reagent capsule 6 can be melted by a first external heater (not shown) during the PCR amplification reaction. Then the fluorescent reagent flows out from the reagent capsule 6 to mix with the amplified product to form the mixed microbead "b", and the mixed microbead "b" enters the electrophoresis box 3 through the outlet 51.

At step four, the electrophoresis box 3 is controlled to perform the electrophoretic process.

In an embodiment, the nucleic acid detection kit 100 is substantially cubic.

In an embodiment, the nucleic acid detection kit 100 is disposable. The nucleic acid detection kit 100 does not need to be re-usable,

The nucleic acid detection kit 100 provided by the present disclosure can integrate the PCR amplification reaction and the electrophoretic process into in a single piece of equipment. The PCR amplification reaction progresses smoothly into the electrophoretic process, which greatly improves the detection efficiency. Thus, the nucleic acid detection kit 100 has a simple structure, which is portable, flexible, and convenient, and can be used at home. At the same time, the detecting process is flexible, and does not need to be done in a professional laboratory. With the above configuration of the electrophoresis box 3, the mixed microbead "b" is easily to move into the electrophoresis box 3 from the channel 5. At the same time, the assembly process of the nucleic acid detection kit 100 is simplified, and the ease of assembly of the nucleic acid detection kit 100 is increased.

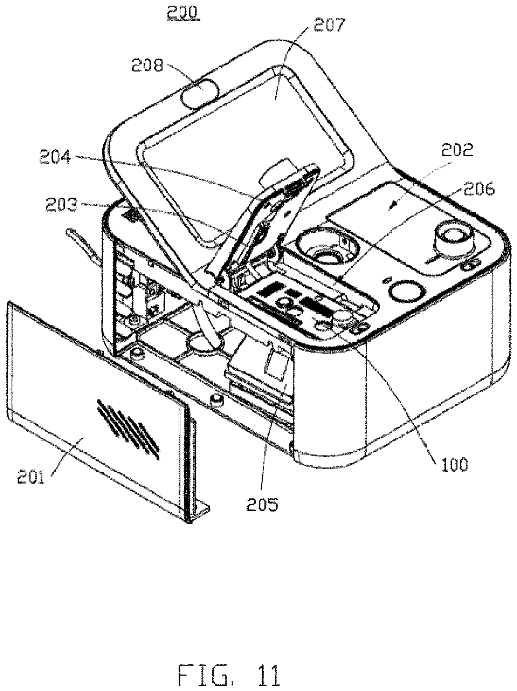

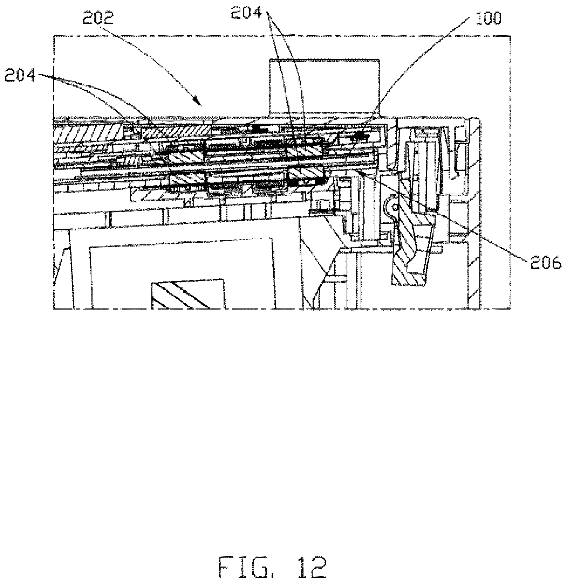

FIG. 11 and 12 illustrate a nucleic acid detection device 200 according to the present disclosure. The nucleic acid detection device 200 includes a host 201 and the nucleic acid detection kit 100. A detection kit mounting area 202 is disposed on the host 201. The nucleic acid detection kit 100 is detachably disposed in the detection kit mounting area 202.

The host 201 further includes a host heating unit 204 and a host connector 203. The host heating unit 204 and the host connector 203 are disposed in the detection kit mounting area 202. When the nucleic acid detection kit 100 is disposed in the detection kit mounting area 202, the host connector 203 can extend into the connecting opening 15 and connect to the control board 4 on the detection chip 2. The host heating unit 204 can extend into the heating hole 14 and connect to the surface of the detection chip 2 for heating the nucleic acid detection kit 100.

In an embodiment, the host heating unit 204 includes the first external heater and the second external heater.

The host 201 further includes the image connection unit 205, which is disposed on a side of the detection kit mounting area 202 corresponding to the electrophoresis box 3. The image connection unit 205 is used to collect the image of fluorescence of the electrophoresis box 3.

Referring to FIG. 12, the detection kit mounting area 202 defines a mounting groove 206. The mounting groove 206 is inclined relative to a top surface of the host 201, which allows oblique placement of the nucleic acid detection kit 100 in the mounting groove 206. Bubbles will be generated during a PCR amplification reaction in the nucleic acid detection kit 100. The bubbles might stay in and block a flow path of microbead "a" in the nucleic acid detection kit 100, so that the microbead "a" would be impeded in the flow path, causing a failure of the nucleic acid detection. Therefore, the mounting groove 206 is designed to be inclined, so that the nucleic acid detection kit 100 can be placed obliquely, and bubbles generated by the PCR amplification reaction are free to disperse without hindering the movement of the microbead.

In an embodiment, the nucleic acid detection device 200 further includes a display screen 207 and a camera 208. The display screen 207 is configured to display an operation interface to allow a user to set operation parameters, and can display the image of fluorescence. The camera 208 is configured to record an operation process of the user, and collect relevant information of the solution (such as information indicating a source of the nucleic acid sample). The host 201 can upload and send the image of fluorescence to a client for relevant personnel to consult.

A nucleic acid sample is mixed with the detection agent (such as a buffer) to form the microbead "a" and is preheated. The microbead "a" enters in the nucleic acid detection kit 100 to undergo the PCR amplification reaction and the electrophoretic process. After the electrophoretic process is completed, the image collection unit 205 acquires the image of fluorescence of the electrophoresis box 3.

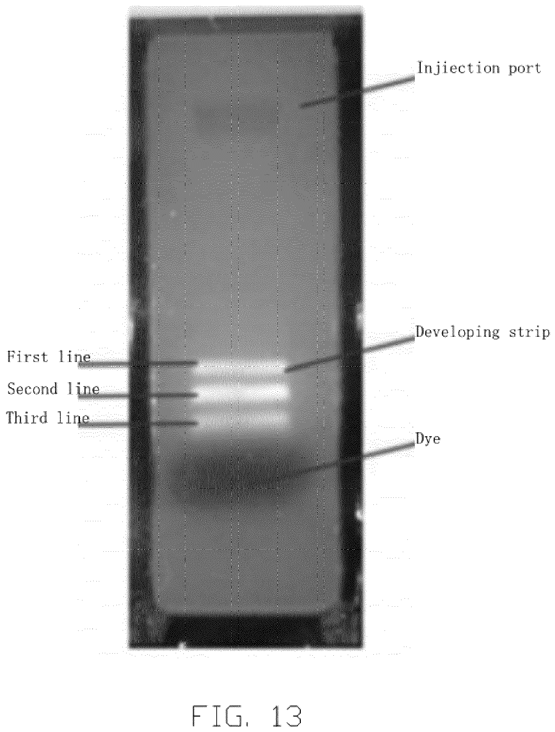

An image of fluorescence of a nucleic acid detection result obtained by the nucleic acid detection device 100 is shown in FIG. 13. In this embodiment, by predefining a range of each line on a standard image of fluorescence, the nucleic acid detection device 100 can automatically identify the nucleic acid detection result when the image of fluorescence is obtained. If a labeling position of a first line on the image of fluorescence is within a predefined range, it can be determined that human genes are included in the nucleic acid sample. If the labeling position of the first line is not within the predefined range, it can be determined that human genes are not included in the nucleic acid sample. If a labeling position of a second line on the image of fluorescence is within the predefined range, it can be determined that RNA replicase is included in the nucleic acid sample. If the labeling position of the second line is not within the predefined range, it can be determined that RNA replicase is not included in the nucleic acid sample. If a labeling position of a third line on the image of fluorescence is within the predefined range, it can be determined that the nucleic acid sample includes N protein. If the labeling position of the third line is not within the predefined range, it can be determined that the nucleic acid sample does not include N protein.

With the above configuration, the nucleic acid detection device 200 provided by the present disclosure integrates the PCR amplification reaction and the electrophoretic process of nucleic acid into in a single equipment through the cooperation of the host 201 and the nucleic acid detection kit 100. Thus, the nucleic acid detection device 200 has a simple structure, which is portable, flexible, and convenient, and can be used at home. At the same time, the detecting process does not need to be carried out in a professional laboratory.

The embodiments shown and described above are only examples. Even though numerous characteristics and advantages of the present technology have been set forth in the foregoing description, together with details of the structure and function of the present disclosure, the disclosure is illustrative only, and changes may be made in the detail, including in matters of shape, size and arrangement of the parts, within the principles of the present disclosure, up to and including, the full extent established by the broad general meaning of the terms used in the claims.

## Claims

1. A nucleic acid detection kit (100), comprising a kit body (1), **characterized in that**, the nucleic acid detection kit (100) further comprises:
a detection chip (2) disposed in the kit body (1); and
an electrophoresis box (3) disposed outside of the kit body (1);
wherein the detection chip (2) comprises a first cover plate (21), a spacer layer (22), and a second cover plate (23), two opposite surfaces of the spacer layer (22) are in contact with the first cover plate (21) and the second cover plate (23), the first cover plate (21), the spacer layer (22), and the second cover plate (23) cooperatively define a channel (5) configured for carrying a microbead (a), the detection chip (2) is connected to the electrophoresis box (3), the microbead (a) undergoes a PCR amplification reaction to obtain a mixed microbead (b) in the channel (5), the mixed microbead (b) undergoes an electrophoretic process in the electrophoresis box (3).

2. The nucleic acid detection kit (100) of claim 1, **characterized in that**, the detection chip (2) further comprises a driving circuit (24), a first dielectric layer (26), a conductive layer (25), and a second dielectric layer (27), the driving circuit (24) is disposed on a surface of the first cover plate (21) close to the second cover plate (27), the first dielectric layer (26) is disposed on a side of the driving circuit (24) close to the second cover plate (27), the conductive layer (25) is disposed on a surface of the second cover plate (23) close to the first cover plate (21), the second dielectric layer (23) is disposed on a side of the conductive layer (25) close to the first cover plate (21), each of the driving circuit (24) and the conductive layer (25) is electrically connected to a power supply, the first dielectric layer (21) and the second dielectric layer (23) cooperatively define the channel (5).

3. The nucleic acid detection kit (100) of claim 2, **characterized in that**, the driving circuit (24) comprises at least one PCR amplification area (C), the kit body (1) defines at least one heating hole (14), the at least one heating hole (14) corresponds to a corresponding one of the at least one PCR amplification area (C), the detection chip (2) exposes through the at least one heating hole (14).

4. The nucleic acid detection kit (100) of claim 3, **characterized in that**, the kit body (1) comprises a first housing (11) and a second housing (12), and each of the first housing (11) and the second housing (12) defines the at least one heating hole (14).

5. The nucleic acid detection kit (100) of claim 2, **characterized in that**, the driving circuit (24) comprises a plurality of driving electrodes (241) disposed in an array, and each of the plurality of driving electrodes (241) is configured to electrically connect to a power supply.

6. The nucleic acid detection kit (100) of claim 5, **characterized in that**, the nucleic acid detection kit (100) further comprises a control board (4), each of the plurality of driving electrodes (241) and the conductive layer (25) are electrically connected to the control board (4), the control board (4) is disposed on a surface of the first cover plate (21) close to the second cover plate (23), and the control board (4) is disposed outside of the channel (5), the kit body (1) further comprises a connecting opening (15), the control board (4) exposes through the connecting opening (15).

7. The nucleic acid detection kit (100) of claim 2, **characterized in that**, the driving circuit (4) further comprises a sample adding area (A), a reagent storage area (B), and a solution outlet area (D), and the solution outlet area (D) is connected to the electrophoresis box (3).

8. The nucleic acid detection kit (100) of claim 7, **characterized in that**, the nucleic acid detection kit (100) further comprises a reagent capsule (6) disposed in the reagent storage area (B), the reagent capsule (6) extends out of the second cover plate (23), the kit body (1) defines a reagent groove (16) corresponding to the reagent storage area (B), and the reagent capsule (6) is received in the reagent groove (16).

9. The nucleic acid detection kit (100) of claim 1, **characterized in that**, the electrophoresis box (3) comprises an electrophoretic body (31), two electrophoretic electrodes (32), and a gel medium (33), the two electrophoretic electrodes (32) are disposed on two ends of the electrophoretic body (31), the two electrophoretic electrodes (32) are opposite to each other, the gel medium (33) is disposed in the electrophoretic body (31), a liquid injection slot (34) is disposed on an end of the get medium (33), each of the two electrophoresis electrodes (32) is configured to electrically connect to a power supply, the first cover plate (21) defines an outlet (51), and the liquid injection slot (34) is connected to the outlet (51).

10. The nucleic acid detection kit (100) of claim 9, **characterized in that**, the nucleic acid detection kit (100) further comprises a first porous adsorption block (38) disposed in the liquid injection slot (34), the first porous adsorption block (38) is configured to carry a wetting liquid (39), one end of the first porous adsorption block (38) extends into a bottom of the liquid injection slot (34), and the other end of the first porous adsorption block (38) extends into the outlet (51), the first porous adsorption block (38) is flush with or protrude from an opening of the outlet (51) closed to the channel (5).

11. The nucleic acid detection kit (100) of claim 9, **characterized in that**, the nucleic acid detection kit (100) further comprises a second porous adsorption block (36) and an adsorption pipe (52), the second porous adsorption block (36) is disposed in the liquid injection slot (34), one end of the adsorption pipe (52) is inserted into the second porous adsorption block (36), and the other end of the adsorption pipe (52) is flush with or protruding from an opening of the outlet (51) closed to the channel (5), the second porous adsorption block (36) is configured to carry a wetting liquid (39), the wetting liquid (39) further extends into and fills the adsorption pipe (52).

12. A nucleic acid detection device (200) comprising a host (201), **characterized in that**, the nucleic acid detection device (200) further comprises:
a nucleic acid detection kit (100) of any one of claims 1 to 11, a detection kit mounting area (202) is disposed on the host (201), the nucleic acid detection kit (100) is detachably disposed in the detection kit mounting area (202).

13. The nucleic acid detection device (200) of claim 12, **characterized in that**, the nucleic acid detection device (200) further comprises a host heating unit (204) and a host connector (203), the host heating unit (204) and the host connector (203) are disposed in the detection kit mounting area (202), each of the detection chip (2) and the electrophoresis box (3) is electrically connected to the host connector (203), the host heating unit (204) is connected to a surface of the detection chip (2) and configured to heat the nucleic acid detection kit (100).
